(19) Europäisches Patentamt
European Patent Office
Office européen des brevets

(11) Veröffentlichungsnummer: **0 184 981**
**B1**

(12) # EUROPÄISCHE PATENTSCHRIFT

(45) Veröffentlichungstag der Patentschrift: **23.08.89**

(51) Int. Cl.⁴: **C 07 D 207/38**

(21) Anmeldenummer: **85810504.2**

(22) Anmeldetag: **01.11.85**

(54) **Neue Pyrrolinone und deren Zwischenprodukte.**

(30) Priorität: **07.11.84 CH 5358/84**

(43) Veröffentlichungstag der Anmeldung: **18.06.86 Patentblatt 86/25**

(45) Bekanntmachung des Hinweises auf die Patenterteilung: **23.08.89 Patentblatt 89/34**

(84) Benannte Vertragsstaaten: **CH DE FR GB IT LI**

(56) Entgegenhaltungen:
**EP-A-0 022 551**
**EP-A-0 094 911**

**Die Akte enthält technische Angaben, die nach dem Eingang der Anmeldung eingereicht wurden und die nicht in dieser Patentschrift enthalten sind.**

(73) Patentinhaber: **CIBA- GEIGY AG, Klybeckstrasse 141, CH- 4002 Basel (CH)**

(72) Erfinder: **Pfenninger, Johannes, Dr., Route du Confin 23, CH- 1723 Marly (CH)**
Erfinder: **Iqbal, Abul, Dr., Im Schaiengarten 1, CH- 4107 Ettingen (CH)**
Erfinder: **Rochat, Alain Claude, Dr., Route de Schiffenen 38, CH- 1700 Fribourg (CH)**

EP 0 184 981 B1

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

LIBER, STOCKHOLM 1989

**Beschreibung**

Aus EP-A-22 551 sind 2-Methyl-carbalkoxypyrrolinone bekannt, die zur Herstellung von 2-Dihalogenmethylen-3-halogen-3-carboalkoxy-5-oxo-pyrrolidinen verwendet werden. Letztere werden als fungizide, bakterizide und algizide Schädlingsbekämpfungsmittel beschrieben.

Es ist nun gefunden worden, dass 2-Aryl-carbalkoxypyrrolinone überraschenderweise wertvolle Zwischenprodukte für die Herstellung von Farbstoffen und Pigmenten, insbesondere von Pyrrolo-[3,4-c]-pyrrolen, darstellen.

Die vorliegende Erfindung betrifft demnach neue Pyrrolinone der Formel

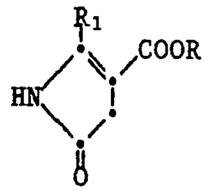

(1),

worin $R_1$ einen Rest der Formel

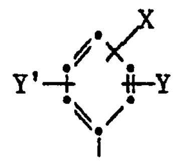

bedeutet, worin X, Y und Y' H- oder Halogenatome, Carbamoyl-, Trifluormethyl-, Cyan- oder $C_2$-$C_6$-Alkylcarbamoylgruppen, Alkyl-, Alkoxy- oder Alkylmercaptogruppen mit 1 - 6 C, Alkoxycarbonyl- oder Alkanoylamino- oder Dialkylaminogruppen mit 2 - 6 C, gegebenenfalls durch Halogen, Alkyl oder Alkoxy mit 1 - 6 C substituierte Phenoxy-, Phenylmercapto, Phenoxycarbonyl-, Phenylcarbamoyl- oder Benzoylaminogruppen bedeuten und mindestens einer der Substituenten X, Y und Y' ein H-Atom bedeutet, und R $C_1$-$C_{18}$-Alkyl, unsubstituiertes oder durch Nitro, Chlor, $C_1$-$C_6$-Alkyl oder $C_1$-$C_6$-Alkoxy substituiertes Phenyl ist.

Bedeutet R in Formel (1) eine $C_1$-$C_{18}$-Alkylgruppe, so kann diese verzweigt oder unverzweigt mit vorzugsweise 1 - 12, und besonders bevorzugt 1 - 4 C-Atomen, sein. Als Beispiel seien Methyl, Äthyl, Isopropyl, sek.- Butyl, tert.-Butyl, tert.-Amyl und Cyclohexyl genannt.

Bedeutet R eine unsubstituierte oder substituierte Phenylgruppe, so ist dies insbesondere unsubstituiertes, oder durch Nitro, Chlor, $C_1$-$C_6$-Alkyl, wie z. B. Methyl, Äthyl, Isopropyl oder tert.-Butyl, oder $C_1$-$C_6$-Alkoxy, wie z. B. Methoxy oder Äthoxy substituiertes Phenyl sein.

$R_1$ steht vorzugsweise für einen Rest der Formel

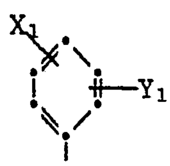

worin einer der Substituenten $X_1$ *und* $Y_1$ ein H-, Chlor- oder Bromatom, eine Methyl-, Cyan-, N,N-Dimethylamino- oder N,N-Diäthylaminogruppe, eine Alkoxygruppe mit 1 - 6 C, eine Alkoxycarbonyl- oder Alkylcarbamoylgruppe mit 2 - 4 C oder eine gegebenenfalls durch Chlor, Methyl oder Methoxy substituierte Phenylcarbamoylgruppe und der andere ein H-Atom bedeuten. Die Substituenten X, $X_1$, Y, Y' und $Y_1$ befinden sich in para-, meta- oder ortho-, vorzugsweise in meta-und para-Stellung zur Pyrrolinon-Gruppe. R stellt bevorzugt $C_1$-$C_{12}$-Alkyl oder Phenyl und vor allem $C_1$-$C_4$-Alkyl dar.

Zu den Pyrrolinonen der Formel (1) gelangt man zum Beispiel durch Cyclisierung eines Aminodicarbonsäureesters der Formel (2)

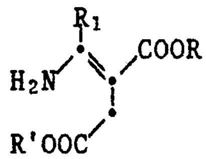

(2),

worin $R_1$ und R die angegebene Bedeutung haben und R' die für R angegebene Bedeutung hat und gleich oder verschieden von R sein kann, mit einer starken Base.

Die Cyclisierung wird in Gegenwart einer starken Base in einem organischen Lösungsmittel durchgeführt.

Geeignete starke Basen sind z. B. Alkalihydroxide, wie Natrium-, Kalium- oder Lithiumhydroxid, oder Erdalkalihydroxide, wie Calcium- oder Magnesiumhydroxid, oder Alkaliamide, wie Lithium- oder Lithiumdiisopropyl-, Lithiumdiäthyl- oder Lithiumisopropyl-cyclohexylamid, Natriumamid, oder Alkalihydride, wie Lithiumhydrid oder Natriumhydrid, oder Erdalkalihydride, wie Calciumhydrid oder Erdalkali- oder Alkalialkoholate, die sich insbesondere von primären, sekundären oder tertiären aliphatischen Alkoholen mit 1 bis 10 C-Atomen ableiten, wie z. B. Natrium-, Kalium-oder Lithiummethylat, -äthylat, -n-propylat, -isopropylat, -n-butylat, -sek.-butylat, -tert.-butylat, -2-methyl-2-butylat, -2-methyl-2-pentylat, -3-methyl-3-pentylat, -3-äthyl-3-pentylat, oder Erdalkali- oder Alkaliphenolate oder o-alkylsubstituierte Phenolate wie Natrium- oder Kalium-o-kresolat. Man kann aber auch ein Gemisch der genannten Basen verwenden.

Man verwendet als starke Basen bevorzugt Alkalialkoholate, wobei Alkali insbesondere Natrium oder Kalium bedeutet, und das Alkoholat sich bevorzugt von einem primären oder sekundären Alkohol ableitet. Besonders bevorzugte starke Basen sind daher z. B. Natrium- oder Kalium-methylat, -äthylat, -isopropylat, -sek.-butylat, -tert.-butylat und -tert.-amylat. Dabei können die Alkalialkoholate auch in situ hergestellt werden, indem man den entsprechenden Alkohol mit dem Alkalimetall, Alkalihydrid oder Alkaliamid umsetzt.

Man setzt die starke Base in einer Menge von insbesondere 0,1 bis 10 Mol, bevorzugt 0,9 bis 4,0 Mol, bezogen auf 1 Mol des Reaktanden der Formel (2) ein.

Als Lösungsmittel eignen sich beispielsweise primäre, sekundäre oder tertiäre Alkohole mit 1 bis 10 C-Atomen, wie Methanol, Äthanol, n-Propanol, Isopropanol, n-Butanol, sek.-Butanol, tert.-Butanol, n-Pentanol, 2-Methyl-2-butanol, 2-Methyl-2-pentanol, 3-Methyl-3-pentanol, 2-Methyl-2-hexanol, 3-Äthyl-3-pentanol, 2,4,4-Trifluormethyl-2-pentanol oder Glykole, wie Äthylenglykol oder Diäthylenglykol, ferner Äther, wie Tetrahydrofuran oder Dioxan, oder Glykoläther, wie Äthylenglykol-methyläther, Äthylenglykol-äthyläther, Diäthylenglykolmonomethyläther oder Diäthylenglykol-monoäthyläther, ferner dipolar-aprotische Lösungsmittel, wie Acetonitril, Benzonitril, Dimethylformamid, N,N-Dimethylacetamid, Nitrobenzol, N-Methyl-pyrrolidon, aliphatische oder aromatische Kohlenwasserstoffe, wie Benzol oder durch Alkyl, Alkoxy oder Halogen substituiertes Benzol, wie Toluol, Xylol, Anisol oder Chorbenzol oder aromatische N-Heterocyclen, wie Pyridin, Picolin oder Chinolin. Die genannten Lösungsmittel können auch als Mischung eingesetzt werden.

Die Umsetzungen werden insbesondere bei einer Temperatur von 20 bis 100°C, vorzugsweise 40 bis 80°C, durchgeführt.

Verwendet man als Lösungsmittel einen Alkohol und als Base ein Alkoholat, so kann es vorteilhaft sein, einen Alkohol und ein Alkoholat mit gleichen Alkylresten zu wählen. Ebenso vorteilhaft kann es sein, wenn zudem noch der Ester der Formel (2) ebensolche Alkylgruppen enthält.

Die Pyrrolinone der Formel (1) erhält man auch nach bekannten Verfahren durch Cyclisierung einer Verbindung der Formel (3)

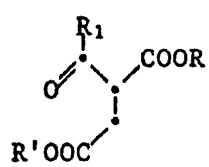

(3)

zum Beispiel mit Ammoniumsalzen.

Die Verbindungen der Formel (3) sind bekannt und können z. B. durch Kondensation eines Acylessigesters der Formel (4)

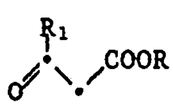

(4),

worin $R_1$ und R die angegebene Bedeutung haben, mit einem Ester der Formel $XCH_2COOR'$, wobei X ein Fluor-, Chlor-, Brom- oder Jodatom bedeutet und R' die angegebene Bedeutung hat, erhalten werden (vgl. W.H. Perkin, J. Chem. Soc. 47, S. 262 oder Org. Synth. 42, 75 (1962)).

Einen weiteren Erfindungsgegenstand bilden die Aminodicarbonsäureester der Formel (2). Diese erhält man

zum Beispiel durch Umsetzen eines Bernsteinsäurediesters der Formel (5)

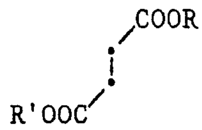

(5)

mit einem Nitril der Formel $R_1CN$, wobei R, R' und $R_1$ die angegebene Bedeutung haben, in Gegenwart einer starken Base und Zink- oder Magnesium-Verbindung analog den in Chem. Lett. 1982, S. 687 und Tetrahedron Lett. 1982, S. 1597 beschriebenen Verfahren.

Bei den zu verwendenden Bernsteinsäurediestern der Formel (5) kann es sich um Dialkyl-, Diaryl-, oder Monoalkylmonoarylester handeln, wobei auch die Bernsteinsäuredialkyl- und -diarylester unsymmetrisch sein können. Bevorzugt verwendet man aber symmetrische Bernsteinsäurediester, insbesondere symmetrische Bernsteinsäuredialkylester. Dabei gelten für Alkyl und Aryl die oben für R und R' angegebene Bedeutungen. Verzweigtes Alkyl ist bevorzugt sek.- oder tert.-Alkyl, wie z. B. Isopropyl, sek.-Butyl, tert.-Butyl oder tert.-Amyl.

Als Beispiele von Bernsteinsäurediestern der Formel (5) seien genannt: Bernsteinsäure-dimethylester, -diäthylester, -dipropylester, -dibutylester, -dipentylester, -dihexylester, -diheptylester, -dioctylester, -diisopropylester, -di-sec.-butylester, -di-tert.-butylester, -di-tert.-amylester, -di-[1,1-dimethylbutyl]-ester, -di-[1,1,3,3-tetramethylbutyl]-ester, -di-[1,1-dimethylpentyl]-ester, -di-[1-methyl-1-äthylbutyl]-ester, -di-[1,1-diäthylpropyl]-ester, -diphenylester, -di-[4-methylpentyl]-ester, -di-[2-methylphenyl]-ester, -di-[4-chlorphenyl]-ester, -monoäthylmonophenylester.

Als Nitrile seien genannt: Benzonitril, o-, m- oder p-Chlorbenzonitril, p-Brombenzonitril, o-, m- oder p-Methylbenzonitril, p-tert.-Butylbenzonitril, p-Methoxybenzonitril, p-Phenoxybenzonitril, 3,4-Dimethylbenzonitril, Isophtalonitril oder Terephthalonitril.

Für obiges Verfahren eigenen sich die gleichen, wie bei der Cyclisierung der Aminodicarbonsäureester der Formel (2) aufgezählten, starken Basen.

Bevorzugt verwendet man als starke Basen Alkaliamide, wobei Alkali insbesonders Lithium bedeutet, und das Amid sich bevorzugt von einem sekundären Amin ableitet. Besonders bevorzugte starke Basen sind daher z. B. Lithium-diisopropyl-, -diäthyl-, oder -isopropyl-cyclohexylamid. Dabei können die Alkaliamide auch in situ hergestellt werden, indem man das entsprechende Amin mit einer Alkalialkylverbindung, dem Alkalimetall, Alkalihydrid oder Alkaliamid umsetzt.

Man setzt die starke Base in einer Menge von insbesondere 0,1 bis 10 Mol, bevorzugt 0,9 bis 4,0 Mol, bezogen auf 1 Mol des Reaktanden der Formel (5) ein.

Die benötigten Zink- und Magnesium-Verbindungen können u.a. als Salze von anorganischen Säuren (z. B. als Halogenide), als Salze von organischen Säuren (z. B. als Acetate), oder aber auch als Alkoholate eingesetzt werden.

Die verwendeten Lösungsmittel sind ebenfalls die gleichen, wie bei der Cyclisierung der Aminodicarbonsäureester der Formel (2) erwähnten Lösungsmittel.

Bevorzugt sind insbesondere solche Lösungsmittel, die gegenüber starken Basen inert sind, nämlich aromatische Kohlenwasserstoffe wie Benzol, durch Alkyl, Alkoxy, oder Halogen substituierte Benzole, oder Äther wie Diäthylähter, Tetrahydrofuran, Dioxan, Anisol, Äthylenglykoldimethyläther oder Diäthylenglykoldimethyläther, ferner heterocyclische aromatische Kohlenwasserstoffe wie Pyridin, Picolin oder Chinolin.

Die Umsetzungen werden insbesondere bei einer Temperatur von 20 bis -100°C, vorzugsweise von -20 bis -80°C durchgeführt.

Die Isolierung der Verbindungen der Formeln (1) und (2) erfolgt zweckmässig durch Hydrolyse des Reaktionsgemisches und Extraktion mit einem organischen Lösungsmittel und Entfernen des letzteren.

Zur Hydrolyse des Kondensationsproduktes kann man eine Säure, einen Alkohol mit 1 bis 4 C-Atomen, wie Methanol oder Äthanol, vorzugsweise aber Wasser, verwenden. Als Säuren kommen z. B. aliphatische oder aromatische Carbon- oder Sulfonsäuren in Betracht, wie beispielsweise Ameisensäure, Essigsäure, Propionsäure, Oxalsäure, Benzoesäure oder Benzolsulfonsäure. Weiterhin kommen als Säuren auch Mineralsäuren in Betracht, wie Chlorwasserstoff, dessen wässerige Lösung, sowie Kohlensäure, verdünnte Schwefel- und Phosphorsäure.

Die Verbindungen der Formeln (1) und (2) stellen wertvolle Zwischenprodukte dar, die sich beispielsweise für die Herstellung von Farbstoffen und Pigmenten eignen, insbesondere für die Herstellung von Pyrrolo-[3,4-c]pyrrolen.

Die folgenden Beispiele erläutern die Erfindung. Temperaturen sind in °C angegeben.

**Beispiel 1:**

In einem durch eine Mischung von Trockeneis und Isopropanol auf -78° gekühlten Reaktionsgefäss werden unter Stickstoffatmosphäre 70 ml wasserfreies Tetrahydrofuran mit 13,8 ml einer 1,6-molaren Lösung n-Butyllithium in Hexan und 3,1 ml Diisopropylamin versetzt. Nach 20 Min. gibt man 2,3 g Bernsteinsäure-di-tert.-butylester in 5 ml Tetrahydrofuran zu und rührt während 50 Min. Dann werden 10 ml einer 1-molaren

Zinkchloridlösung in Tetrahydrofuran zugefügt und nach 30 Min. wird das Reaktionsgemisch mit 2,1 g Benzonitril versetzt. Nach 2 Std. lässt man auf Raumtemperatur erwärmen und giesst das Reaktionsgemisch auf 200 ml Wasser. Das erhaltene Gemisch wird in Essigester aufgenommen, die organische Phase mit einer konz. Kochsalzlösung gewaschen, mit Natriumsulfat getrocknet und das Lösungsmittel am Rotationsverdampfer entfernt. Durch Chromatographie an Kieselgel mit Hexan/Essigester 8 : 1 erhält man 1,96 g (59 % d. Th., bezogen auf den Bernsteinsäure-di-t-butylester) der Verbindung der Formel (6)

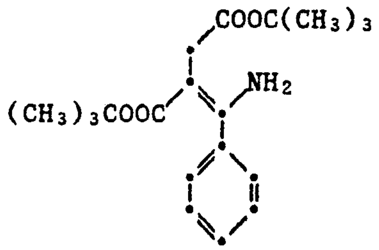

(6)

oder des entsprechend Z-Isomeren als farblose Kristalle.

Smp.: 112 - 114°, UV (MeOH, $\lambda_{max}$, ε): 222(6750), 288(13750).

| | | | | |
|---|---|---|---|---|
| $C_{19}H_{27}NO_4$ | Ber.: | C 68,44 | H 8,16 | N 4,20 |
| | Gef.: | C 68,37 | H 8,16 | N 4,21. |

**Beispiel 2:**

Man verfährt wie im Beispiel 1, setzt aber anstelle von Benzonitril 4-Chlorbenzonitril ein. Das Produkt der Formel (7)

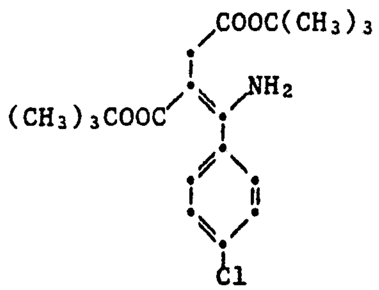

(7)

oder das entsprechende Z-Isomere wird in 68-%-iger Ausbeute, bezogen auf Bernsteinsäure-di-tert.-butylester, isoliert.

Smp.: 123 - 124°; UV (MeOH, $\lambda_{max}$, ε): 222(10200) 288(12900).

| | | | | | |
|---|---|---|---|---|---|
| $C_{19}H_{26}NO_4Cl$ | Ber.: | C 62,03 | H 7,12 | N 3,81 | Cl 9,64 |
| | Gef.: | C 62,08 | H 7,15 | N 3,75 | Cl 9,70. |

**Beispiel 3:**

5,03 g der gemäss Beispiel 1 erhaltenen Verbindung der Formel (6) (oder des entsprechenden Z-Isomeren) werden mit 60 ml Methanol und 30 ml 30 % Natriummethylat in Methanol versetzt und unter Stickstoffatmosphäre während 40 Min. auf 60° erwärmt.

Man giesst das Reaktionsgemisch auf Essigester, neutralisiert mit 1N Salzsäure und wäscht mit konz. Kochsalzlösung. Die organische Phase wird mit Natriumsulfat getrocknet und am Rotationsverdampfer eingeengt. Durch Chromatographieren an Kieselgel mit Toluol/Essigester 4 : 1 erhält man 2,34 g (60 % der Theorie) der kristallinen Verbindung der Formel (8)

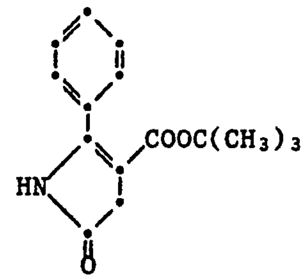

(8)

vom Smp. 153 - 154°.

| | | | | |
|---|---|---|---|---|
| $C_{15}5H_{17}NO_3$ | Ber.: | C 69,48 | H 6,61 | N 5,40 |
| | Gef.: | C 69,22 | H 6,61 | N 5,32. |

**Beispiel 4:**

100 g Benzoylbernsteinsäurediäthylester und 111 g Ammoniumacetat werden in 300 ml Eisessig während 16 Stunden am Rückfluss gekocht. Das Reaktionsgemisch wird auf 3 Liter kaltes Wasser gegossen. Es bildet sich ein kristalliner Niederschlag, der abgenutscht und mit 500 ml Wasser gewaschen wird. Das Rohprodukt wird in Methylenchlorid umkristallisiert und man isoliert 48,9 g Kristalle der Formel (9)

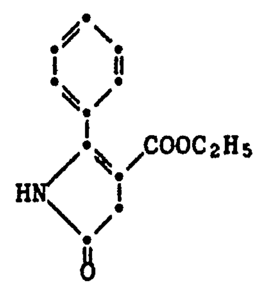

(9)

Smp.: 174°.

| | | | | |
|---|---|---|---|---|
| $C_{13}H_{13}NO_3$ | Ber.: | C 67,52 | H 5,67 | N 6,06 |
| | Gef.: | C 67,00 | H 5,71 | N 5,97. |

**Beispiel 5:**

1,55 g Natrium und 0,02 g Sulfobernsteinsäure-bis-2-äthylhexylester-Natriumsalz (Emulgator) werden bei Rückflusstemperatur in 27 ml tert.-Amylalkohol bis zur vollständigen Umsetzung gerührt. Zur klaren Lösung gibt man bei 100° 6 g 4-Chlorbenzonitril und anschliessend während 30 Min. portionsweise 5,1 g des gemäss Beispiel 4 erhaltenen Pyrrolinons der Formel (9).

Das Reaktionsgemisch wird eine Stunde bei 100° gerührt und auf 200 ml kaltes Wasser ausgegossen. Man lässt eine Stunde bei Rückflusstemperatur rühren und leitet anschliessend zur Entfernung des organischen Lösungsmittels während einer Stunde Wasserdampf ein. Die Pigmentsuspension wird filtriert und der Filterkuchen im Vakuum bei 80° getrocknet. Man erhält 7,2 g [74 %] Pigment der Formel (10),

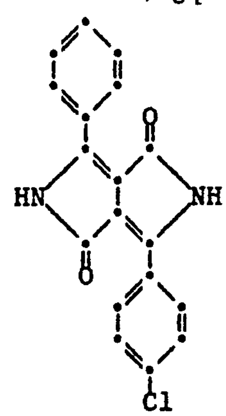

(10),

das, in PVC eingearbeitet, eine rote Färbung ergibt.

Absorption im sichtbaren Bereich/in NMP, $\lambda_{max}$ ε): 471 (25300), 510 (34100)

| | | | | |
|---|---|---|---|---|
| $C_{18}H_{11}N_2O_2Cl$: | Ber.: | C 66,98 | H 3,44 | N 8,68 |
| | Gef.: | C 66,92 | H 3,60 | N 8,54. |

**Beispiel 6:**

Man verfährt wie in Beispiel 1, setzt aber anstelle von Bernsteinsäuredi-tert.-butylester Bernsteinsäurediisopropylester ein.

Das nach der Extraktion erhaltene ölige Rohprodukt wird in 1,5 % Natriummethoxid in Methanol 30 Min. bei 60° erwärmt. Anschliessend lässt man auf Zimmertemperatur abkühlen, nimmt in Essigester auf, neutralisiert mit 1N Salzsäure und wäscht mit konz. Kochsalzlösung neutral. Nach dem Trocknen mit Natriumsulfat, Filtrieren und Einengen isoliert man das Produkt der Formel

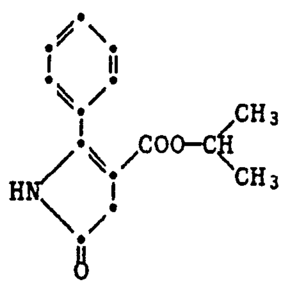

in einer Ausbeute von 39 % bezogen auf Bernsteinsäurediisopropylester. Eine Probe, aus Methylenchlorid /Hexan umkristallisiert, schmilzt bei 148 - 150°.

| | | | | |
|---|---|---|---|---|
| $C_{14}H_{15}NO_3$ | Ber.: | C 68,56 | H 6,16 | N 5,71 |
| | Gef.: | C 68,45 | H 6,25 | N 5,75 |

**Beispiel 7:**

Man geht gleich vor wie in Beispiel 1, setzt aber statt Benzonitril Terephthalonitril ein.

Nach Chromatographie an Kieselgel mit Toluol Essigester 4 : 1 werden 1,81 g (5 % d.Th., bezogen auf den Bersteinsäure-di-tert.-butylester) der Verbindung der Formel

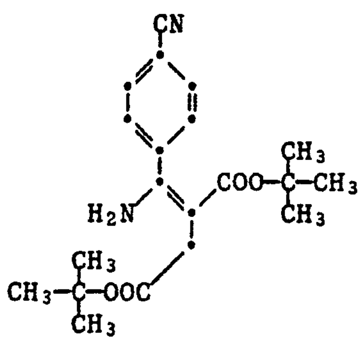

oder des entsprechenden Z-Isomeren als farblose Kristalle isoliert.

Smp. 158 - 159°; UV (MeOH $\lambda_{max}$, ε) 230(16500), 265(11400), 305(sh).

| | | | | |
|---|---|---|---|---|
| $C_{20}H_{26}N_2O_4$ | Ber.: | C 67,02 | H 7,31 | N 7,82 |
| | Gef.: | C 67,03 | H 7,39 | N 7,74 |

**Beispiel 8:**

Man verfährt wie in Beispiel 1, setzt aber statt Benzonitril Isophthalonitril ein. Das Produkt der Formel

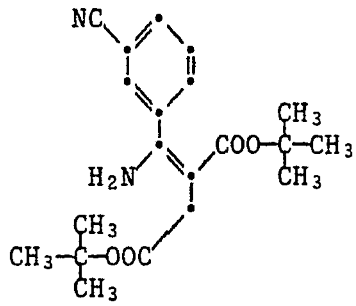

oder das entsprechende Z-Isomere kann in einer Ausbeute von 62,3 % isoliert werden.
Smp. 112 - 114°; UV (MeOH $\lambda_{max}$, ε) 225(14200), 275(sh), 288(9600).

| | | | | |
|---|---|---|---|---|
| $C_{20}H_{26}N_2O_4$ | Ber.: | C 67,02 | H 7,31 | N 7,82 |
| | Gef.: | C 67,43 | H 7,25 | N 8,56. |

**Beispiel 9:**

22,7 g der Verbindung der Formel

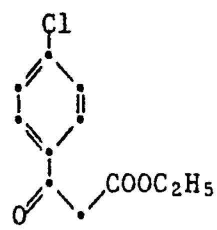

13,4 g Chloressigester und 15,2 g Kaliumcarbonat-Pulver werden in 60 ml Aceton und 40 ml Dimethoxyäthan während 22 Std. am Rückfluss gekocht. Man lässt auf Raumtemperatur abkühlen, filtriert und wäscht mit Hexan. Das Filtrat wird am Rotationsverdampfer eingeengt und man isoliert in praktisch quantitativer Ausbeute die Verbindung der Formel

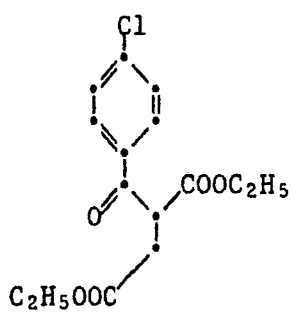

die als Rohprodukt direkt weiterverwendet wird. Das Rohprodukt und 78,7 g Ammoniumacetat werden in 90 ml Eisessig während 2 1/2 Std. am Rückfluss gekocht. Das Gemisch wird auf Eis/Wasser ausgegossen und das ausgefällte Rohprodukt wird abfiltriert. Umkristallisieren in Äthanol/Wasser liefert 16 g (60 % d. Th.) kristallines Produkt der Formel

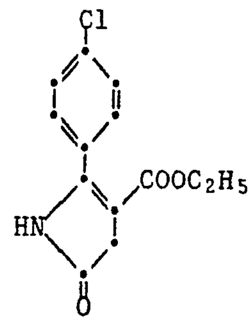

Smp. 195 - 196°.

| | | | | | |
|---|---|---|---|---|---|
| $C_{13}H_{12}NO_3Cl$ | Ber.: | C 58,77 | H 4,55 | N 5,27 | Cl 13,34 |
| | Gef.: | C 58,77 | H 4,52 | N 5,20 | Cl 13,37. |

**Patentansprüche**

1. Pyrrolinone der Formel

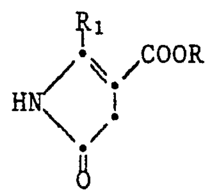

,

worin $R_1$ einen Rest der Formel

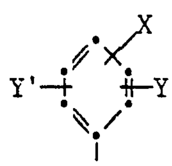

bedeutet, worin X, Y und Y' H- oder Halogenatome, Carbamoyl-, Trifluormethyl-, Cyan- oder $C_2$-$C_6$-Alkylcarbamoylgruppen, Alkyl-, Alkoxy- oder Alkylmercaptogruppen mit 1 - 6 C, Alkoxycarbonyl- oder Alkanoylamino- oder Dialkylaminogruppen mit 2 - 6 C, gegebenenfalls durch Halogen, Alkyl oder Alkoxy mit 1 - 6 C substituierte Phenoxy-, Phenylmercapto, Phenoxycarbonyl-, Phenylcarbamoyl- oder Benzoylaminogruppen bedeuten und mindestens einer der Substituenten X, Y und Y' ein H-Atom bedeutet, und R $C_1$-$C_{18}$-Alkyl, unsubstituiertes oder durch Nitro, Chlor, $C_1$-$C_6$-Alkyl oder $C_1$-$C_6$-Alkoxy substituiertes Phenyl ist.

2. Pyrrolinone gemäss Anspruch 1, worin $R_1$ einen Rest der Formel

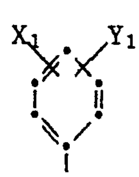

bedeutet, worin einer der Substituenten $X_1$ und $Y_1$ ein H-, Chlor- oder Bromatom, eine Methyl-, Cyan-, N,N-Dimethylamino- oder N,N-Diäthylaminogruppe, eine Alkoxygruppe mit 1 - 6 C, eine Alkoxycarbonyl- oder Alkylcarbamoylgruppe mit 2 - 4 C oder eine gegebenenfalls durch Chlor, Methyl oder Methoxy substituierte Phenylcarbamoylgruppe und der andere ein H-Atom bedeuten, und R $C_1$-$C_{12}$-Alkyl oder Phenyl ist.

3. Pyrrolinone gemäss Anspruch 1, worin R eine $C_1$-$C_4$-Alkylgruppe bedeutet.

4. Verfahren zur Herstellung der Pyrrolinone gemäss Anspruch 1, dadurch gekennzeichnet, dass man einen Aminodicarbonsäureester der Formel

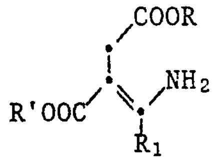

worin $R_1$ die in Anspruch 1 angegebene Bedeutung hat, R und R' unabhängig voneinander $C_1$-$C_{18}$-Alkyl, unsubstituiertes oder durch Nitro, Chlor, $C_1$-$C_6$-Alkyl oder $C_1$-$C_6$-Alkoxy substituiertes Phenyl bedeuten, in Gegenwart einer starken Base in einem organischen Lösungsmittel umsetzt.

5. Verfahren gemäss Anspruch 4, dadurch gekennzeichnet, dass man als Base ein Alkalialkoholat verwendet.

6. Verfahren gemäss Anspruch 4, dadurch gekennzeichnet, dass man als Lösungsmittel einen Alkohol verwendet.

7. Aminodicarbonsäureester der Formel

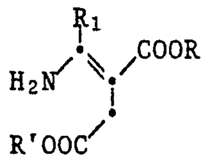

worin R und $R_1$ die im Anspruch 1 angegebene Bedeutung haben und R' die für R angegebene Bedeutung hat und gleich oder verschieden von R sein kann.

8. Aminodicarbonsäureester gemäss Anspruch 7, worin $R_1$ die im Anspruch 2 angegebene Bedeutung hat und R und R' unabhängig voneinander $C_1$-$C_4$-Alkyl bedeuten.

9. Verfahren zur Herstellung der Aminodicarbonsäureester gemäss Anspruch 7, dadurch gekennzeichnet, dass man einen Bernsteinsäurediester der Formel

$R'OOCCH_2CH_2COOR$

mit einem Nitril der Formel $R_1CN$ in Gegenwart einer starken Base und Zinkchlorid umsetzt, wobei R, R' und $R_1$ die in Anspruch 7 angegebene Bedeutung haben.

10. Verfahren gemäss Anspruch 9, dadurch gekennzeichnet, dass man als starke Base ein Alkaliamid verwendet.

11. Aminodicarbonsäureester gemäss Anspruch 7, worin R und R' $C_1$-$C_4$-Alkylgruppen bedeuten.

## Claims

1. A pyrrolinone of the formula

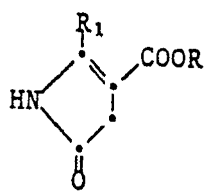

in which $R_1$ is a radical of the formula

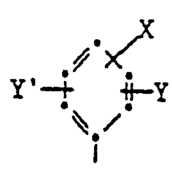

in which X, Y and $Y^1$ are hydrogen or halogen atoms, carbamoyl, trifluoromethyl, cyano, $C_2$-$C_6$alkylcarbamoyl groups, alkyl, alkoxy or alkylmercapto groups of 1 - 6 C, alkoxycarbonyl or alkanoylamino or dialkylamimo groups of 2 - 6 C, phenoxy, phenylmercapto, phenoxycarbonyl, phenylcarbamoyl or benzoylamino groups

which are unsubstituted or substituted by halogem, alkyl or alkoxy of 1 - 6 C, at least one of the substituents X, Y and Y' being a hydrogen atom, and R is $C_1$-$C_{18}$alkyl or phenyl which is unsubstituted or substituted by nitro, chlorine, $C_1$-$C_6$-alkyl or $C_1$-$C_6$alkoxy.

2. A pyrrolinone according to claim 1, in which $R_1$ is a radical of the formula

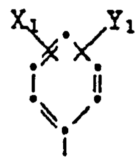

in which one of the substituents $X_1$ and $Y_1$ is a hydrogen, chlorine or bromine atom, a methyl, cyano, N,N-dimethylamino or N,N-diethylamino group, an alkoxy group of 1 - 6 C, an alkoxycarbonyl or alkylcarbamoyl group of 2 - 4 C, or a phenylcarbamoyl group which is unsubstituted or substituted by chlorine, methyl or methoxy, and the other substituent is a hydrogen atom, and R is $C_1$-$C_{12}$alkyl or phenyl.

3. A pyrrolinone according to claim 1, in which R is a $C_1$-$C_4$alkyl group.

4. A process for the preparation of a pyrrolinone according to claim 1, which comprises reacting an aminodicarboxylic ester of the formula

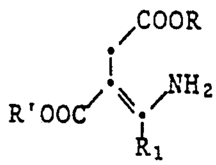 ,

in which $R_1$ is as defined in claim 1 and each of R and R' independently of the other is $C_1$-$C_{18}$alkyl or phenyl which is unsubstituted or substituted by nitro, chlorine, $C_1$-$C_6$alkyl or $C_1$-$C_6$alkoxy, in the presence of a strong base in an organic solvent.

5. A process according to claim 4, wherein the base used is an alkali metal alcoholate.

6. A process according to claim 4, wherein the solvent used is an alcohol.

7. An aminodicarboxylic ester of the formula

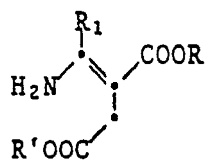 ,

in which R and $R_1$ are as defined in claim 1 and R' has the meanings given for R and may be identical to or different from R.

8. An aminodicarboxylic ester according to claim 7, in which $R_1$ is as defined in claim 2 and each of R and R' independently of the other is $C_1$-$C_4$alkyl.

9. A process for the preparation of the aminodicarboxylic esters according to claim 7, which comprises reacting a succinic diester of the formula

$R'OOCCH_2CH_2COOR$

with a nitrile of the formula $R_1CN$, in which R, R' and $R_1$ are as defined in claim 7, in the presence of a strong base and zinc chloride.

10. A process according to claim 9, wherein the strong base used is an alkali metal amide.

11. An aminodicarboxylic ester according to claim 7, in which R and R' are $C_1$-$C_4$alkyl groups.

**Revendications**

1. Pyrrolinones répondant à la formule:

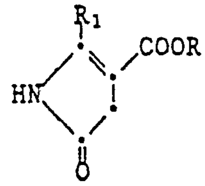

dans laquelle:

$R_1$ représente un radical répondant à la formule :

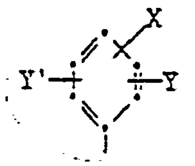

dans laquelle X, Y et Y' représentent chacun un atome d'hydrogène ou d'halogène, un carbamoyle, un trifluorométhyle, un cyano, un alkylcarbamoyle en $C_2$-$C_6$, un radical alkyle, alcoxy ou alkylthio en $C_1$-$C_6$, un radical alcoxycarbonyle, alcanoylamino ou dialkylamino en $C_2$-$C_6$, un radical phénoxy, phénylthio, phénoxycarbonyle, phénylcarbamoyle ou benzoylamino éventuellement porteur d'un halogène ou d'un radical alkyle ou alcoxy en $C_1$-$C_6$, et au moins l'un des symboles X, Y et Y' représente un atome d'hydrogène, et

R représente un alkyle en $C_1$-$C_{18}$, ou un radical phényle non substitué ou porteur d'un nitro, d'un chlore, d'un alkyle en $C_1$-$C_6$ ou d'un alcoxy en $C_1$-$C_6$.

2. Pyrrolinones selon la revendication 1 dans lesquelles $R_1$ représente un radical répondant à la formule :

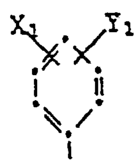

dans laquelle l'un des symboles $X_1$ et $Y_1$ représente un atome d'hydrogène, de chlore ou de brome, un radical méthyle, cyano, N,N-diméthylamino ou N,N-diéthylamino, un radical alcoxy en $C_1$-$C_6$, un radical alcoxycarbonyle ou alkylcarbamoyle en $C_2$-$C_4$ ou un radical phénylcarbamoyle éventuellement porteur d'un chlore, d'un méthyle ou d'un méthoxy, et l'autre représente un atome d'hydrogène,

et R représente un alkyle en $C_1$-$C_{12}$ ou un phényle.

3. Pyrrolinones selon la revendication 1 dans lesquelles R représente un alkyle en $C_1$-$C_4$.

4. Procédé de préparation des pyrrolinones selon la revendication 1, procédé caractérisé en ce qu'on fait réagir un ester d'acide amino-dicarboxylique répondant à la formule:

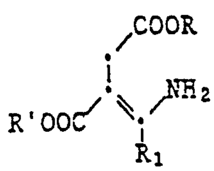

dans laquelle $R_1$ a la signification qui lui a été donnée à la revendication 1, et R et R' représentent chacun, indépendamment l'un de l'autre, un alkyle en $C_1$-$C_{18}$, un phényle non substitué ou un phényle porteur d'un nitro, d'un chlore, d'un alkyle en $C_1$-$C_6$ ou d'un alcoxy en $C_1$-$C_6$,

en présence d'une base forte, dans un solvant organique.

5. Procédé selon la revendication 4 caractérisé en ce qu'on utilise, comme base, un alcoolate de métal alcalin.

6. Procédé selon la revendication 4 caractérisé en ce qu'on utilise, comme solvant, un alcool.

7. Esters d'acides amino-dicarboxyliques répondant à la formule:

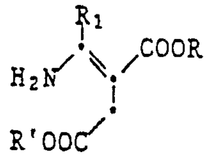

dans laquelle R et $R_1$ ont les significations qui leur ont été données à la revendication 1 et R' a la signification qui a été donnée pour R et peut être identique à R ou différent de lui.

8. Esters d'acides amino-dicarboxyliques selon la revendication 7 dans lesquels $R_1$ a la signification qui lui a été donnée à la revendication 2, et R et R' représentent chacun, indépendamment l'un de l'autre, un alkyle en $C_1$-$C_4$.

9. Procédé pour préparer les esters d'acides amino-dicarboxyliques selon la revendication 7, procédé caractérisé en ce qu'on fait réagir un diester de l'acide succinique de formule:

$R'OOCCH_2CH_2COOR$

avec un nitrile de formule $R_1CN$, formules dans lesquelles R, R' et $R_1$ ont les significations qui leur ont été données à la revendication 7, en présence d'une base forte et de chlorure de zinc.

10. Procédé selon la revendication 9 caractérisé en ce qu'on utilise, comme base forte, un amidure de métal alcalin.

11. Esters d'acides amino-dicarboxyliques selon la revendication 7 dans lesquels R et R' représentent chacun un radical alkyle contenant de 1 à 4 atomes de carbone.